# EUROPEAN PATENT SPECIFICATION

(11) **EP 3 328 334 B1**
(45) Date of publication and mention of the grant of the patent: **07.04.2021**
(21) Application number: 16763571.3
(22) Date of filing: 27.07.2016
(51) Int. Cl.: A61F 9/008, A61F 9/009

(54) **REMOTELY-CONTROLLED APPARATUS FOR OPHTHALMIC SURGERY**
FERNGESTEUERTE VORRICHTUNG FÜR DIE AUGENCHIRURGIE
APPAREIL COMMANDÉ À DISTANCE POUR LA CHIRURGIE OPHTALMIQUE

(30) Priority: 29.07.2015 IT UB20152609
(43) Date of publication of application: 06.06.2018
(73) Proprietor: Ghinelli, Emiliano, 00144 Roma (IT)
(72) Inventor: Ghinelli, Emiliano, 00144 Roma (IT)
(74) Representative: Pes, Matteo
(86) International application number: PCT/IB2016/054472
(87) International publication number: WO 2017/017616

(56) References cited:
- US-A1- 2009 182 310

## Description

### Field of the Invention

The present invention relates to an apparatus for remotely carrying out eye diagnosis and surgery operations, i.e. with the surgeon far from the patient.

### State of the art

Recently in the field of the ophthalmic surgery, robotic apparatuses have been proposed allowing the surgeon operating even remotely on a patient's eye, i.e. from a remote position, with modes which fall within the so-called "telemedicine".

For example US 2010/0331858 describes a remotely-controlled apparatus for applying micro-stent in blood vessels of a patient. The apparatus can be constrained to the patient's head and is provided with robotic arms remotely controlled by the surgeon by means of an interface comprising a joystick and a monitor on which the images taken from the surgical site are displayed. The arms are provided with relative actuators; the surgeon drives the actuators through the interface to obtain the desired movements by the arms. Clearly, the surgical instruments needed to complete the operation are assembled on board to the arms.

US 2013/338679 teaches making a robotic apparatus comprising a single arm and having a four-bar linkage geometry. The surgical instrument is assembled on the arm and is able to translate with respect to it. The surgeon remotely controls the apparatus.

The use of robotic apparatuses is also known in other fields of medicine, as described for example in WO 99/58055, US 9060793, US 2015/0164597, US 2015/0157411, WO 2012/037506, but in general these are solutions that cannot be used for the ophthalmic surgery, because the human eye considerably differs from the other organs in size, structure, geometry and features.

Currently available solutions suffer from some drawbacks.

The surgeon imparts commands to the actuators of the apparatus to obtain corresponding movements of the robotic arms and surgical instruments. Therefore, mistakes made by the surgeon are reflected on the movement of the surgical instruments and cause mistakes in the operation on the patient's eye. Since the surgeon is remotely working, by observing a display, easily he can be misled if the patient's eye is moving during the operation. Thus, the good outcome of surgical operations also depends on the ability of the surgeon to properly interpret what he sees on the display and compensate possible undesirable movements of the patient's eye. In other words the apparatuses available at present still depend a lot on the cleverness of the surgeon, whereas it is desirable to have solutions that allow minimizing or cancelling the risk of mistakes in his interpretation of the images.

Also, another drawback of the solutions available at present is related to the ability of the surgeon: possible mistakes made by the surgeon in operating the actuators of the robotic arms are reflected on the movement of the surgical instruments. Thus, the human error cannot be excluded. It is desirable instead to have apparatuses aiding the surgeon or entirely replacing him in the highest possible number of maneuvers of the robotic arms and instruments connected to the latter.

Still another limitation of the current robotic apparatuses is constituted by the fact that all of the components and also the environment in which they operate must be sterilized. The sterilization of the components of the robotic apparatuses must be carried out prior to each operation. Therefore, known apparatuses are subjected to long moments of inactivity between an operation and the following one.

US 2009/182310 describes a system for collimating a laser or X-ray beam on the patient's eye. The system comprises a structure supporting the patient's head and an adjustable arm on which a foot is assembled for being positioned on the patient's eye, to stabilize its orientation in the space. Once the foot is adherent to the eye, the relative orientation in space is determined with respect to the reference system of the supporting structure; by conveniently orienting an emitter, it is possible to collimate a beam in a specific region of the eye to carry out a radiation or laser therapy (par. 11). The foot is substantially concave, in order to be shaped as the ocular surface also with the aid of a slight depression, between 20 and 50 Hg, caused by specific means in fluid communication with the foot. The position and the orientation of the foot in the space are detected in this way: on the foot there are light sources emitting light beams and the system comprises corresponding receivers. The direction of the beams (trigonometry) uniquely determines the position and the orientation of the foot. Thus, the laser/radiotherapic emitter can be consequently oriented. The position of the arm is manually or automatically adjustable (par. 85). Also the position of the laser/X-ray emitter is itself manually or automatically adjustable, even remotely adjustable, with servomotors (par. 82).

### Summary of the Invention

Therefore, the object of the present invention is to provide an apparatus that allows solving the drawbacks of the known solutions.

Therefore, the present invention concerns an apparatus according to claim 1.

In particular, the apparatus is distinguished from the known art by comprising at least one robotic arm in which a surgical instrument is telescopically housed. The arm is provided with a foot leaning on the patient's eye, for example on the cornea, and with suction means keeping, if needed, the foot adherent to the patient's eye, for example as the surgical instrument is inserted in the anterior chamber of the eye.

In this way a closed operating system is accomplished, that does not necessitate the use of the operating room. The environment between the foot of the robotic arm and the patient's eye remains isolated from the surrounding environment. The robotic arm and the relative surgical instrument are sterile and therefore the risk of causing infections is avoided.

Also the insertion of the surgical instruments, in addition to the relative following movements, are remotely controlled and the apparatus helps the surgeon, so obtaining a minimization of the risks of human error in the movements.

The system described in US 2009/182310 doesn't provide surgical instruments telescopically inserted in the adjustable arm; thus it is not a system adapted for remotely carrying out operations of surgery, but only a system for the collimation of laser or X-rays.

The proprietor of the present Patent Application would like to call the apparatus with the name of OSIRIS, acronym of *Ophthalmic Surgery Intelligent Robotic Integrated System.*

Preferably the apparatus comprises means for directing a laminar flow of clean and filtered air on the surgical site. This allows the operators to move around the patient, if needed, keeping sterile the surgical site.

In a second aspect thereof, for which the Applicant reserves the right to file a Divisional Patent Application, the present invention relates to a container for ocular tissues intended for the transplantation. The container is provided with retaining means that keep the ocular tissue stationary with respect to the container itself. The lid of the container can be sealed, to guarantee sterility, and at the same time it acts as an applanation surface and is transparent to laser. In this way, the surgeon can operate on the ocular tissue with the laser prior to the transplantation, but without withdrawing the tissue from the container.

### Brief List of Figures

Further characteristics and advantages of the invention will be more evident by the review of the following specification of a preferred, but not exclusive, embodiment depicted for illustration purposes only and without limitation, with the aid of the attached drawings, in which:
- figure 1 is a schematic view of the ocular anatomy;
- figure 2 is a schematic view of a first optional component of the apparatus according to the present invention;
- figure 3 is a schematic view of a second component of the apparatus according to the present invention;
- figure 4a is a schematic top view of an eye subjected to surgical operation with the apparatus according to the present invention;
- figure 4b is a schematic view of a third component of the apparatus according to the present invention, in use during a surgical operation on the eye shown in figure 4a;
- figure 5 is a schematic view of the third component of the apparatus according to the present invention, in use during a surgical operation on the eye shown in figure 4a;
- figure 6 is a schematic view of the apparatus according to the present invention;
- figure 7 is a schematic view of some details of the apparatus according to the present invention
- figure 8 is a schematic top view of the patient's eye and a component of the apparatus according to the present invention, during a surgical operation;
- figure 9 comprises two schematic views, from top and laterally, of the patient's eye and a component of the apparatus according to the present invention, during a surgical operation;
- figure 10 is a schematic view of a work station of the apparatus according to the present invention;
- figure 11 is a view of an attachment of the apparatus according to the present invention;
- figure 12 is a schematic view of the surgeon remotely operating by using a dummy to impart commands;
- figure 13 is a partial section view of a container of ocular tissue, intended for the transplantation, according to a second aspect of the present invention;
- figure 14 is a partial view of the container of figure 13, during the use.

### Detailed Description of the Invention

The following description refers to an embodiment of the apparatus according to the present invention applied to the surgery of the anterior segment of the human eye, and particularly used for remotely carrying out a cataract operation.

However, in general the apparatus can also be used to carry out refractive surgical operations, with or without implantation of intraocular lenses, vitreoretinal surgery, operations of cornea transplantation or reconstruction of the anterior segment, etc.

In principle, the apparatus according to the present invention can also be used for other operations of neurological or vascular microsurgery, i.e. in other sectors of the medicine.

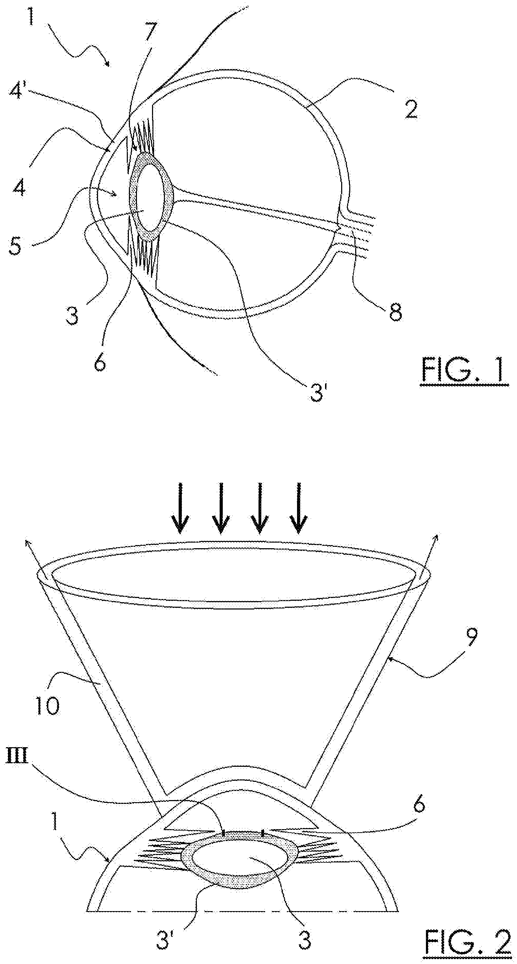

Figure 1 schematically shows the anatomy of a human eye. The eyeball 1 comprises a rear portion 2, that is behind the crystalline lens 3, and a front portion 4, that is in front of the crystalline lens 3. In turn, the front portion 4 comprises an anterior chamber 5 placed in front of the iris 6, and a rear chamber 7 placed between the iris 6 and the crystalline lens 3. The optic nerve 8 transmits the signals to the brain.

The crystalline lens 3 is contained in a capsule of tissue that, in cataract operations, must be incised by the surgeon just to be able to remove the crystalline lens 3 to be replaced by an artificial lens.

Figure 2 schematically shows an optional component 9 of the apparatus according to the present invention. It is an applanation lens 9 leant on front portion of the patient's eye 1, particularly on the cornea. The lens 9 has conical shape specifically conceived to focus the laser beams in a spot with a diameter of about 2-3 microns.

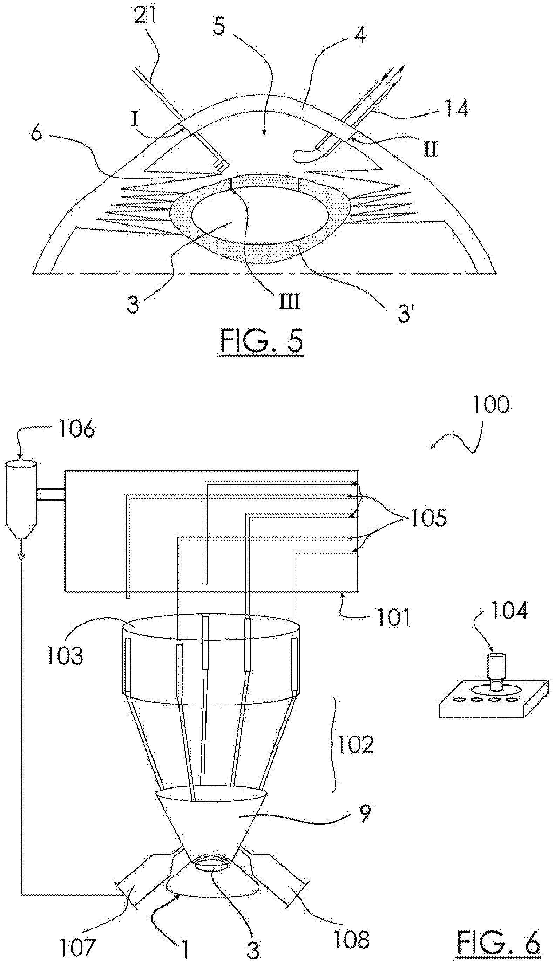

The applanation lens 9 is moved to abutment against the cornea 4' of the patient by means of a supporting structure (shown in figure 6) of the apparatus. The adhesion of the applanation lens 9 to the cornea 4' is obtained by creating a slight suction on the cornea 4' itself, by sucking air by means of a pump (not shown) connected to channels 10 obtained in the thickness of the applanation lens 9 itself. The channels 10 open at the lower surface of the applanation lens 9, the same surface abutting against the cornea 4'.

The applanation lens 9 is connected to an image capturing system (not shown), for example a high definition 3D camera and an OCT. Preferably the applanation lens 9 is itself part of the image capturing system.

The images are transmitted to a monitor 11 that can also be placed far from the patient. The surgeon observes the images on the monitor 11 and uses them to operate a laser, for example a femtosecond laser commonly known as femtolaser, or else a nanolaser. In particular the surgeon sees, frontally and laterally, the eye surface on the monitor 11, also thanks to the computerized optical tomography allowing a view of the profile of ocular tissues.

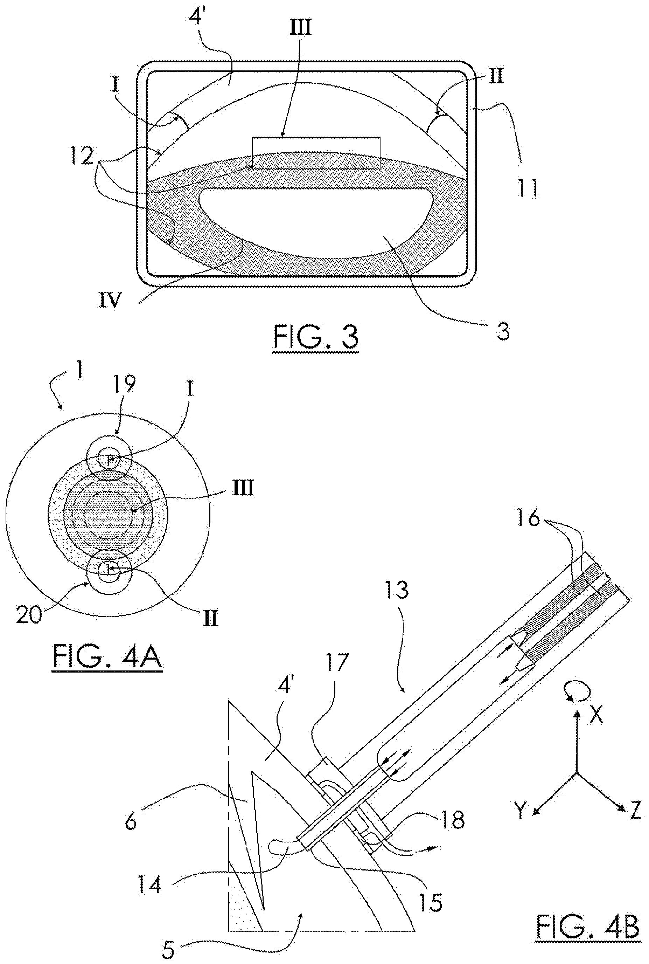

Figure 3 is a purely schematic view of images transmitted on the display 11 by the capturing system. A computer analyzes the images and assists the surgeon in the choice of the areas of the eye 1 intended to be hit with the laser beams passing through the applanation lens 9; these beams are schematically depicted in figure 2 with harrows pointing downwards.

A plurality of lines 12 are superimposed on the images transmitted by the monitor 11. Such lines 12 define the pattern of the cuts the surgeon will carry out in the eye tissue by using the femtolaser and identify different areas of the eye 1. For example, the cuts I and II in the corneal tissue serve to allow the insertion of the surgical instruments in the front chamber 5, the circular cut III is the capsulotomy, i.e. a circular opening created on the front surface of the capsule 3' of the crystalline lens 3, and the area IV correspond to the crystalline lens 3 to be fragmented by the laser, extracted from the eye 1 and replaced with an artificial lens.

In astigmatic patients two cuts (not shown) are carried out in the outer edge of the cornea 4' in order to correct the refractive defect.

At this point, the applanation lens 9 can be moved away from the eye 1 to make room for the surgical instruments; it could be used again, if needed.

Alternatively, the applanation lens 9 is left coupled to the eye 1 and the surgical instruments are housed inside it and can be remotely moved by the surgeon.

Figure 4A shows the eye 1 from top as it would be seen through the applanation lens 9 adherent to the cornea 4', as shown in figure 2. In this step the iris 6 of the patient has been dilated with a drug so that it doesn't obstruct the access to the crystalline lens 3. The line III, the most inner one, corresponds to the circular cut the surgeon made on the anterior capsule of the crystalline lens 6 by means of the laser. The cuts I and II are also visible.

The apparatus according to the present invention is distinguished from the prior art in that it comprises at least one robotic arm 13 schematically shown in figure 4B, that can be remotely controlled by the surgeon and able to adhere to the eye 1. Preferably, the apparatus comprises more than one robotic arm.

The robotic arm 13 can be translated on the three axes X, Y and Z and can be rotated on the X axis, and preferably also on the Y and Z axes. The robotic arm 13 is supported by a frame (not shown in figure 4B) designed to follow the movements of the patient's eye, i.e. to modify the position of the robotic arm 13 so that the eye 1 and the arm 13 itself are moving substantially together with no variation of the mutual position.

At least one surgical instrument 14 is installed inside the robotic arm 13; the surgeon arranges in advance the instruments 14 that he must use during the operation. For example, the surgeon can opt to design the apparatus with four robotic arms 13, each equipped with a corresponding surgical instrument 14, a laparoscope, etc.

The surgical instrument 14 can slide in the robotic arm 13 so that to be telescopic and partially extractable from the end 15 of the arm itself. The surgical instrument 14 is controlled by pull rods 16 operated by apposite actuators, for example electric engines, in turn remotely controlled by the surgeon.

To guarantee optimal vision a medical fluid is fed, if needed, through the surgical instrument, for example an emulsifier or else a balanced saline solution.

Advantageously, the robotic arm 14 comprises a leaning foot 17 having the function of moving to abutment against the surface of the patient's eye 1; in the example shown in figure 4B the foot 17 is in abutment against the cornea 4'. The adhesion of the foot 17 to the eye 1 is guaranteed by a suction effect caused by the controlled air sucking by a plurality of channels 18 which open at the surface of the foot 17 facing towards the cornea 4'. It is just this suction to keep the robotic arm 13 in contact with the eye 1 for the time needed to allow inserting the surgical instrument 14 through the corneal wall. In other words, the force generated by the air suction by the channels 18 is sufficient to keep the foot 17 adherent on the cornea 4 even when the surgical instrument 14 is facing resistance in passing through the wall of the cornea 4'; to avoid damages to the eye, i.e. to avoid the collapsing of the chambers, in every circumstance the apparatus must guarantee the equivalence between the inner pressure of the eye 1 and the tractive force applied by the foot 17 by virtue of the sucking (equivalence of the pressures). Thus, the foot 17 allows obtaining, for all the time needed, the coupling of the robotic arm 13 to the eye 1.

Preferably, the apparatus comprises one or more proximity sensors generating signals indicative of the distance of the leaning foot 17 or the surgical instrument from the patient's eye.

The proposed solution is also advantageous because it allows isolating the surgical site with respect to the surrounding environment and this allows intervening on the eye 1 also in environments different than the traditional operating room. In particular, the closed environment between the surface of the eye 1 and the foot 17 doesn't run the risk of bacterial contaminations because the robotic arm 14 is sterile.

Preferably the apparatus comprises two robotic arms 13. With the reference numbers 19 and 20 in figure 4A the perimeters of the feet 17 of the arms 13, that are in abutment against the cornea 4' just above the cuts I and II previously carried out by the laser, are depicted. The surgical instruments 14 are inserted in the anterior chamber 5 through the cuts I and II.

Preferably, the apparatus according to the present invention comprises a computerized controlling unit provided with software for recognizing the images. The software and the CPU are able to interface to all of the variables coming into play during the surgery and, thanks to the artificial intelligence (for example a neural network), they are able to suggest to the surgeon the best patterns of execution, or else to block the surgeon if he is about to carry out a movement outside the patterns. The software analyzes and "learns" the best operational strategies and "communicates" with all of the current and future components of the apparatus (Osiris). The software identifies the cuts I and II made by the laser through the corneal wall and automatically controls the movement of the robotic arms 13 to lead the respective feet 17 in correct alignment with such cuts. This function can be defined *auto-docking* of the robotic arms.

In particular, the surgical instrument 14 shown in figure 4B is a phacoemulsifier, in essence an ultrasonic probe breaking in small pieces the core of the crystalline lens 3 that has already been fragmented by the laser beams. The fragments are removed by a sucking line inside the phacoemulsifier 14 itself.

The cataract operation continues.

Figure 5 is a schematic view of the patient's eye 1 during the operation, in a moment subsequent to that shown in figures 4A and 4B. The robotic arm 13 is drawn back, leaving the surgical instrument 14 inserted in the anterior chamber 5.

Another surgical instrument 21 serving to remove the front surface of the previously cut capsule 3' and so to communicate with the crystalline lens 3, is introduced in the chamber 5 by a corresponding robotic arm (not shown) equivalent to the arm 13.

Figure 5 shows a moment when both the robotic arms have been drawn back, thus leaving the instruments 14 and 21 in the anterior chamber 5 of the eye 1; the surgeon remotely moves the instruments 14 and 21, thanks to the supporting structure mentioned above and the actuators.

With the phacoemulsifier 14 inserted through the cut III, the surgeon removes the core of the crystalline lens 3.

At this point another robotic arm is moved to abutment against the eye 1 in order to provide an apposite instrument allowing the surgeon to insert an artificial lens inside the capsule 3'.

All of the surgical instruments are removed from the eye 1 and the operation is almost finished.

At this point the surgeon proceeds to practice a hydrosuture, consisting in the infiltration of physiological solution at the edges of the cut III in order to bring the surfaces closer and seal them together avoiding the application of the classical point of suture.

At the discretion of the surgeon a solution containing antibiotic is injected into the anterior chamber 5.

Figure 6 is a schematic and overall view of an apparatus 100 according to the present invention for the remotely-controlled ophthalmic surgery. Such an apparatus 100 cannot provide for the applanation system shown in figure 2.

The apparatus 100 comprises a main body 101 on which the head of the femtosecond or nanosecond laser (not visible) and a structure 102 for the support and movement of the robotic arms 13 are assembled, in which the surgical instruments 14, 21 and the instruments of the applanation lens 9 are housed.

As previously described, the supporting structure 102 can be oriented in the space to follow the movements of the patient's eye.

The supporting structure 102 can be coupled and de-coupled to/from the main body 101 by a connecting member 103, that is ring-shaped in the example shown. The connecting ring 103 is interchangeable. This feature is particularly useful in the surgical field because it allows guaranteeing the sterility of parts that can come in contact with the patient. The applanation lens 9, that is optional, the supporting structure 102 and the connecting ring 103 are replaced at each new surgical operation and from time to time sterile-packed, as well as the surgical instruments the surgeon chooses to arrange in advance in the robotic arms.

The surgical instruments needed for the operation are loaded in advance in the apparatus 100 by the surgeon or his staff.

In this way, the bacterial translocation towards the patient's eye is avoided.

In the embodiment shown in figure 6, the robotic arms 13 are slidingly housed in the thickness of the applanation lens 9. In an alternative embodiment, not shown, the robotic arms 13 are outside the applanation lens 9 and free in the space above the operating table.

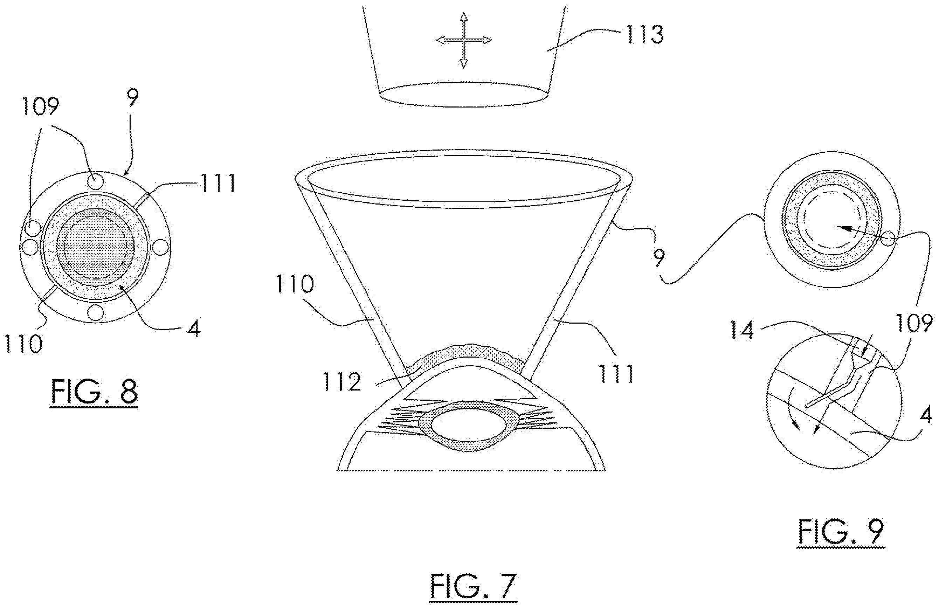

In an embodiment the surgical instruments are housed in apposite channels, for example five, obtained in the wall of the applanation lens 9 and more clearly visible in figure 8.

Thus, in general, the robotic arms 13 can be directly constrained to the supporting structure 102 and/or the applanation lens 9, the latter being in turn supported by the structure 102. Thus, the robotic arms 13 can independently reach the surgical site thanks to the "auto-docking" function, i.e. computer driven auto-positioning.

The applanation lens 9 is adhered to the cornea 4 of the patient's eye 1, as explained above, just by means of the supporting structure 102. The surgeon remotely controls the movements of the supporting structure 102, for example by means of a joystick 104, a pad, a work station for virtual/increased reality, etc.

The pipes 105, feeding the medical liquids or gasses to the surgical instruments, are housed in the main body 101. The connecting ring 103 can therefore be sealingly connected to the main body 101 to give continuity to the pipes 105.

With the reference number 106 is depicted a distribution system of balanced saline solution; it is a solution applied to the ocular surface to guarantee a correct vision and avoid damages of the eye surface. Two connectors 107 and 108 are linked to the applanation lens 9 at horizontal inner ducts of the lens 9, in order to allow circulating the saline solution. Alternatively, the saline solution can also flow inside the telescopic arms and, in this case, the ducts circulating the saline solution are linked to the arms.

Figure 7 shows the applanation lens 9 placed on the cornea 4 and the head of the femtolaser or nanolaser 113 approaching on the applanation lens 9. The saline solution 112 is injected through the horizontal inlet duct 110, then discharged by duct 111.

Figure 8 schematically shows the applanation lens 9 viewed from top, and the auto-docking pattern of the robotic arms 13, i.e. the position where the suckers of the arms 13 will applanate, when the lens is placed on the cornea 4 of a patient. The channels 109 obtained from the thickness of the lens 9 serve to house the robotic arms 13 or directly the surgical instruments. The horizontal ducts 110 and 111, and alternatively the duct 109 of the robotic arm 13, serve the circulation of the saline solution, and practically must be engaged by the connectors 107 and 108 or free in case it is the duct 109 to circulate the saline solution.

Figure 9 shows in detail a surgical instrument 14 that is pushed in the relative channel 109, freely or by use of the applanation lens 9, towards the cornea 4. The movement is remotely controlled by the surgeon, by the joystick 104. A medical fluid is fed parallel to the instrument 14.

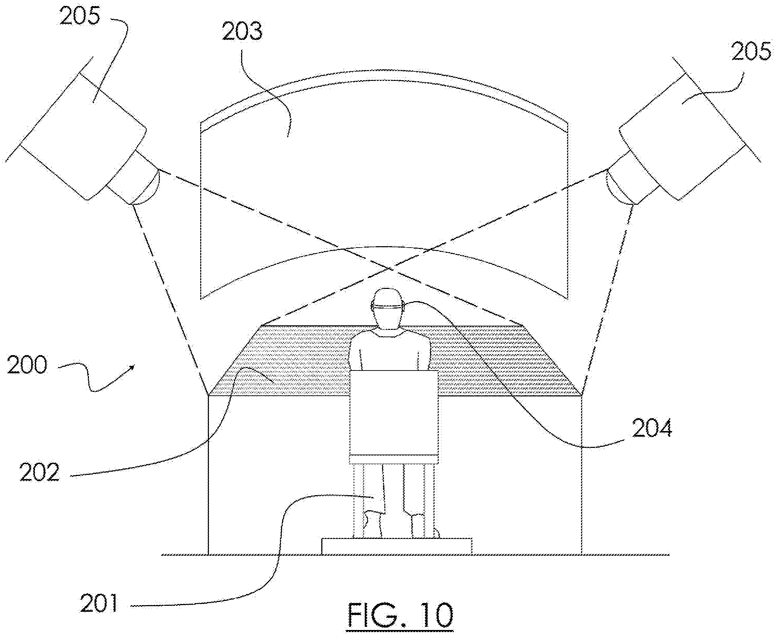

Figure 10 shows the remote work station 200 of the surgeon 201. This work station is an alternative to the control by the joystick 104, particularly useful for the surgical simulation and the training. The surgeon is in front of a working surface 202 and the display 203. Preferably, the display 203 transmits 3D images and the surgeon is provided with apposite glasses 204 for the 3D vision.

In an embodiment the glasses 204 interact with the surgeon, meaning that they detect the relative movements of the eyes and, based on these movements, they transmit commands to the apparatus. Practically the glasses 204 themselves work as a command instrument.

The surgeon 201 can partially drive the monitor 203 through pedals (not visible in figure), for example he can rotate the transmitted images or else can switch from a full-screen view to a parallel-screen view, etc.

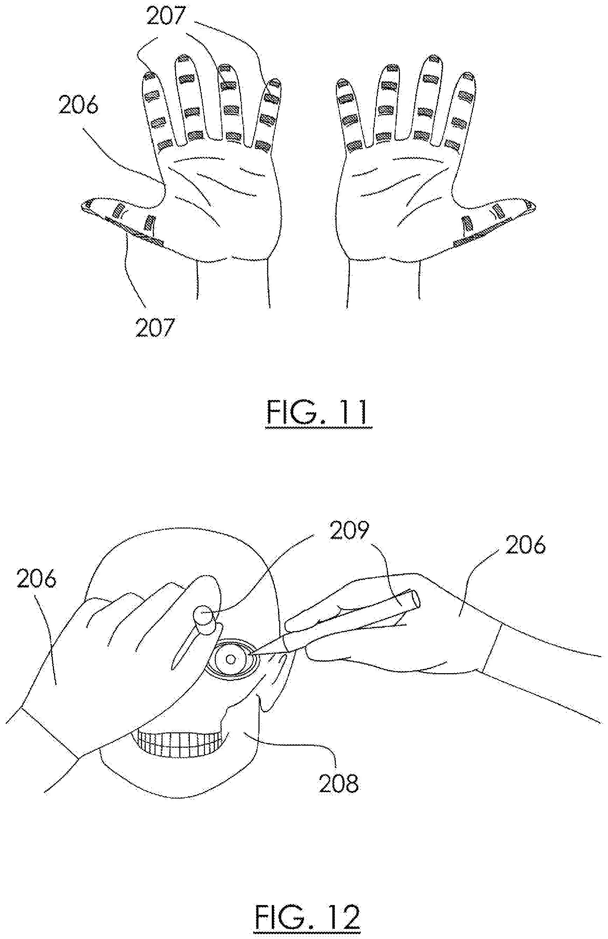

The working surface 202 is sensitive to electrical pulses generated by apposite sensors assembled on the gloves 206 worn by the surgeon 201 and shown in detail in figure 11. For example, the working surface 202 can be of capacitive type. The cameras 205 film the working surface 202. Practically, a processor processes the signals received from the working surface 202 and cameras 205 to determine the displacements made by the surgeon's 201 single finger and hands and the position the fingers and hands are taking in the space instant by instant, and to drive the apparatus 100 in a corresponding way to carry out the surgical operation on the patient's eye.

Figure 11 shows in detail the gloves 206. The gloves are equipped with a plurality of sensors 207 each generating an electrical signal proportional to the pressure locally applied on the sensor itself. The work station 200 is designed to calculate the distance of each sensor 207 from the working surface 202.

For example, the sensors 207 are pressure switches with variable resistance.

As shown in figure 12, the surgeon can simulate the remote operation by using a dummy 208 placed on the working surface 202, by using false surgical instruments 209 and the gloves 206. Each movement in the space imparted to the instruments 209 is immediately repeated by the apparatus 100 by means of the actuators, motors, etc., so that the surgical instruments 14 exactly repeat the same movements with respect to the patient's eye. Practically, the supporting structure 102 is oriented in the space in order to lead the surgical instruments 14 to the correct orientation with respect to the eye and just to properly move the instruments 14, with the same pressure applied by the surgeon on the instruments 209. The surgeon continuously observes through the display 203 what is happening in the surgical site.

A controlling unit (not shown) detects the signals of the gloves 206, working surface 202 and cameras 205 provided by the sensors 207, and uses them to controls the displacements of the robotic arms (13).

The controlling unit works with a software preferably structured as a neural network memorizing and analyzing each movement imparted by the surgeon in each operation, so that the neural network develops over time self-cognitive capabilities, identification capabilities of possible case studies and self-learning capabilities, to suggest the subsequent moves to the surgeon, possibly by proposing the best-judged movements, cuts and patterns as superimposed on the display, just depending on the analyses performed on the previous cases. In this way the controlling unit assists the surgeon in his choices, and is designed as an assistance that provides experience-based suggestions in real time.

Optionally, the apparatus comprises means (not shown in the drawings) for constantly directing a laminar flow of clean and filtered air onto the surgical site, at the patient's eye or at the foot of the robotic arm. Such means can comprise, for example, a low-speed blower provided with filters and a settling chamber to ease the laminar flow. When the surgical site is hit by a laminar flow of clean air, the risks of contamination are minimal.

Preferably, fastenings used in the positioning process are similar to those used in the trocar.

Preferably, the apparatus also comprises an optical fiber for the endoillumination.

Preferably, the apparatus is provided with sensors detecting the instrument temperature and/or the vibrations to which the instruments are subjected or to which the tissues of the patient's eye are subjected, for example because of the heartbeat or the movements caused by the breathing. The signals generated by such sensors are collected and processed by the controlling unit, for possible signaling to the surgeon.

Preferably, the apparatus is provided with sensors detecting the vicinity of the instruments to ocular structures and generating corresponding signals processed by the controlling unit to provide corresponding information to the surgeon.

Preferably, the apparatus is also provided with at least one thermographic camera remotely detecting the surface temperature of the patient's eye and of the single ocular tissues subjected to the operation.

Preferably the apparatus is also provided with sensors, for example strain gauges, conveniently arranged on the robotic arm in order to detect its tensional state, for example to detect if it is subjected to bending and to what extent. The signals generated by the sensors are processed by the controlling unit that, if needed, based on the comparison with threshold values, send alarm signals to the operating surgeon. Preferably, the strain gauges are optical ones of the FBG type.

If needed, the apparatus also comprises sensors detecting the deformation of the tissues of the patient's eye, for example sensors capturing photographic images and processing them to detect any dimensional change.

If needed, the apparatus also comprises sensors detecting the transparency of the tissues and sensors measuring the energy release and absorption of the tissues.

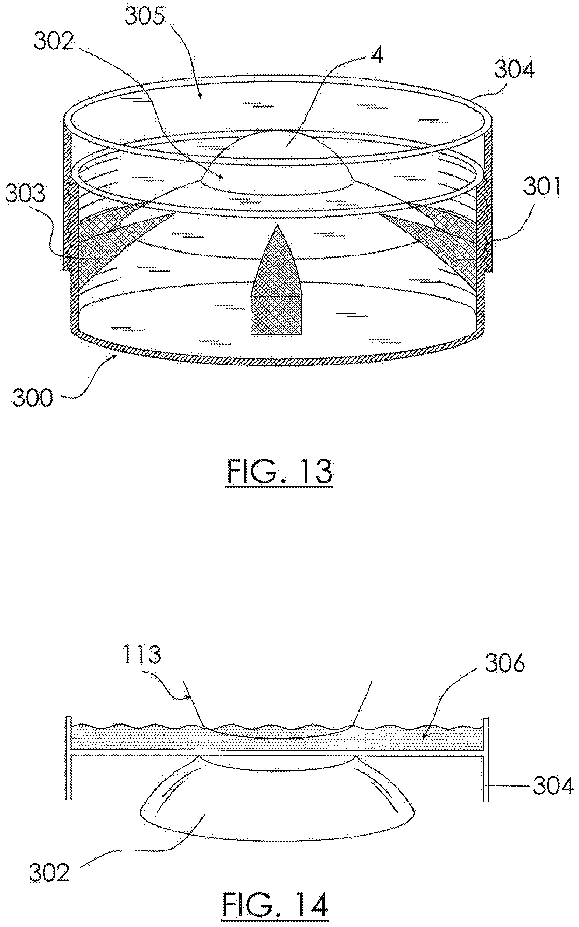

Figure 13 shows an optional aspect of the present invention for which the Applicant reserves the right to file a divisional patent application. It is a container 300 of ocular tissues intended for the transplantation. The container 300 is filled with a culture medium in which a sample of the ocular tissue 302 taken from a donor is immersed. The ocular tissue 302 is retained by a plurality of tweezers 303, for example fastening strips, or else a supporting surface, for example a kind of grid. In this way the tissue 302 is kept properly stretched.

The container 300 is closed by a lid 304, for example screwed as shown in figure 13. The seal is tight, to guarantee that the culture medium could not leak and the tissue could not be contaminated. The upper surface 305 of the lid 304 is itself designed as an applanation surface in the sense that, by screwing the lid 304 on the container 300, it can be moved to abutment against the ocular tissue 302 as the applanation lens 9 would do.

The surface 305 of the lid 304 can be constituted by, for example, a membrane such as the "ultrapore" membranes used in cytology.

The surface 305 of the lid 304 is transparent to the laser beams produced by the femtolaser head 113.

Thus, the environment inside the container 300 is kept sterile and the tissue can easily be transported where the receiver is.

The big advantage offered by the container 300 is that the preparation of the ocular tissue 302 for the transplantation can largely be directly made in the container itself, and thus still in sterile conditions.

Figure 14 shows an example. The lid 304 has been screwed on the container 300 up to a height sufficient to press on the ocular tissue 302, thus partially flattening it. The head 113 of the femtolaser or nanolaser is moved to abutment on the lid 304 with the interposition, or not, of a viscoelastic medium 306. At this point, the surgeon can operate with the laser on the tissue 302 without taking it out of the container 300. Advantageously, the ocular tissue 302 can be prepared for the transplantation on the patient with no need to take it out of the container 300.

## Claims

1. An apparatus (100) for remotely carrying out operations of ophthalmic surgery, comprising:
- a laser emitter (113),
- surgical instruments (14, 21),
- a supporting structure (102) able to position the laser emitter (113) with respect to the patient's eye (1), and
- remote controlling means (104, 200, 206, 208, 209) to remotely control the surgical instruments (14, 21) and the laser emitter (113),
- at least one robotic arm (13) remotely controlled as well, in which the surgical instrument (14) is telescopically housed and
in that the robotic arm (13) is provided with a foot (17) leaning on the patient's eye (1) and with suction means (18) keeping, if need be, the foot (17) adherent to the patient's eye (1), for example as the surgical instrument (14) is inserted in the anterior chamber (5) of the eye (1).

2. Apparatus according to claim 1, wherein the foot (17) leant on the eye (1) defines, together with the surface (1) of the eye itself, a site isolated from the surrounding environment and in itself adapted to carry out surgical operations.

3. Apparatus according to claim 1 or claim 2, further comprising an applanation lens (9) and wherein the supporting structure (102) is designed to position the applanation lens (9) on the patient's eye (1) and the laser emitter (113) with respect to the applanation lens (9).

4. Apparatus according to any one of claims 1-3, wherein the suction means (18) comprise channels which open at the foot (17) and are connected to a sucking pump, which is preferably calibrated so that the suction remains balanced with respect to the inner pressure of the eye (1), so that the eye (1) does not deform and is not subjected to damages.

5. Apparatus according to any one of claims 1-4, wherein pull rods (16) adapted to impart movements to the surgical instrument (14), and/or feeding duct to feed medical fluids to the surgical instrument (14), and/or sucking ducts to suck ocular tissues, for example tissues of a crystalline lens (3) disintegrated by the laser, are housed in the robotic arm (13).

6. Apparatus according to any one of preceding claims 1-5, wherein the robotic arm (13) can be retracted so that the surgical instrument (14) can be left in the eye (1) properly.

7. Apparatus according to any one of preceding claims 1-6, further comprising a circuit (106, 107, 108) for circulating balanced saline solution at the eye (1), wherein the applanation lens (9), if present, is part of said circuit, being provided with apposite channels (110, 111).

8. Apparatus according to any one of preceding claims 3-7, wherein the applanation lens (9) is sustained by the supporting structure (102) that, in turn, is coupled to a main body (101) in which the laser emitter is housed (113), by means of a connecting member (103), so that the assembly formed by the applanation lens (9), the supporting structure (102) and the connecting member (103) can be released from the main body (101) of the apparatus and sterilized before every operation.

9. Apparatus according to claim 8, wherein the connecting member is provided with channels and connectors in order to fluidically communicate the main body (101), in which feeding ducts to feed medical gases or sucking ducts are housed, with the surgical instruments (14, 21).

10. Apparatus according to any one of preceding claims 1-9, wherein the remote controlling means comprise a display (203) on which images of the eye (1) are projected, and controlling instruments of joystick type (104).

11. Apparatus according to any one of preceding claims 1-9, wherein the remote controlling means comprise:
- a display (203) on which images of the eye (1) are projected, gloves (206) the surgeon can wear and provided with pressure sensors (207), false surgical instruments (209) the surgeon handles with the gloves (206), a dummy (208) simulating the patient's eye, and
- a working surface (202) combined with sensors selected among proximity sensors of the foot with respect to the tissues of the patient's eye, deformation sensors of the tissues, temperature sensors of the tissues, transparency sensors of the tissues and energy release and absorption sensors of the tissues, and
- cameras to detect the position of the surgeon's hands and fingers with respect to the dummy (208), wherein the pressure sensors (207) detect the pressure the surgeon applied onto false surgical instruments (209), and
- a controlling unit processing the signals provided by said sensors and the cameras of the working surface in order to impart the movements to the robotic arms (13) and the surgical instruments (14, 21).

12. Apparatus according to any one of preceding claims 1-11, comprising a container (300) of ocular tissues (302) intended for the transplantation, wherein the container (300) can be filled with a culture medium and in turn comprises:
- a plurality of tweezers (303) designed to retain the ocular tissue (302), or else a supporting surface that performs the same function, so that it cannot move with respect to the container itself, and
- a lid (304) for sealingly closing the container (300) so that the inner environment thereof remains sterile,
wherein the lid (304) also acts as an applanation surface and is transparent to laser beams in order to allow the surgeon to prepare the ocular tissue (302) for the transplantation without opening the container (300), so that contaminations can be prevented.

13. Apparatus according to any one of preceding claims 1-12, comprising means for directing a laminar flow of clean and filtered air on the surgical site, at the patient's eye or at the foot of the robotic arm.

14. Apparatus according to any one of preceding claims 1-13, comprising at least one optical fiber for the endoillumination of the surgical site.

15. Apparatus according to any one of preceding claims 1-14, comprising sensors detecting the instrument temperature and/or the vibrations to which the instruments are subjected.

## Patentansprüche

1. Ein Apparat (100) zur ferngesteuerte Durchführung von Operationen für die ophthalmische Chirurgie, umfassend:
- einen Laseremitter (113),
- chirurgische Instrumente (14, 21),
- eine Stützstruktur (102), die in der Lage ist, den Laseremitter (113) bezüglich des Patientenauges (1) zu positionieren, und
- Steuermittel (104, 200, 206, 208, 209) zur Fernsteuerung der chirurgischen Instrumente (14, 21) und des Laseremitters (113),
- wenigstens einen ebenfalls ferngesteuerten Roboterarm (13), in dem das chirurgische Instrument (14) teleskopartig aufgenommen ist und
wobei der Roboterarm (13) mit einem auf das Patientenauge (1) lehnenden Füßchen (17) und mit Saugmitteln (18) versehen ist, die, wenn nötig, das Füßchen (17) an dem Patientenauge (1) haftend halten, beispielsweise wenn das chirurgische Instrument (14) in die vordere Kammer (5) des Auges (1) eingesetzt ist.

2. Apparat nach Anspruch 1, wobei das am Auge (1) anliegende Füßchen (17) zusammen mit der Oberfläche (1) des Auges selbst eine von der Umgebung isolierte Stelle definiert, die ihrerseits zur Durchführung chirurgischer Operationen geeignet ist.

3. Apparat nach Anspruch 1 oder 2, ferner umfassend eine Applanationslinse (9) und wobei die Stützstruktur (102) dazu ausgebildet ist, die Applanationslinse (9) auf das Patientenauge (1) und den Laseremitter (113) bezüglich der Applanationslinse (9) zu positionieren.

4. Apparat nach einem der Ansprüche 1 bis 3, **dadurch gekennzeichnet, dass** die Saugmittel (18) beim Füßchen (17) sich öffnende Kanäle aufweisen, die mit einer Saugpumpe verbunden sind, die vorzugsweise so kalibriert ist, dass die Saugwirkung gegenüber dem Innendruck des Auges (1) ausgeglichen bleibt, so dass sich das Auge (1) nicht verformt und keiner Beschädigung unterworfen ist.

5. Apparat nach einem der Ansprüche 1 bis 4, wobei Zugstangen (16), die dazu eingerichtet sind, dem chirurgischen Instrument (14) Bewegungen zu vermitteln, und/oder Zuführkanäle zum Zuführen von medizinischen Flüssigkeiten zu dem chirurgischen Instrument (14), und/oder Saugkanäle zum Ansaugen von Okulargeweben, beispielsweise Gewebe einer durch den Laser desintegrierten Augenlinse (3), in dem Roboterarm (13) untergebracht sind.

6. Apparat nach einem der vorhergehenden Ansprüche 1 bis 5, wobei .der Roboterarm (13) derart zurückgezogen werden kann, um das chirurgische Instrument (14) korrekterweise im Auge (1) eingesetzt zu belassen.

7. Apparat nach einem der vorhergehenden Ansprüche 1 bis 6, ferner umfassend einen Kreislauf (106, 107, 108) zum Umwälzen einer ausgeglichenen Salzlösung im Bereich des Auges (1), wobei die Applanationslinse (9), falls vorhanden, Teil des Kreislaufes ist und mit passenden Kanälen (110, 111) versehen ist.

8. Apparat nach einem der vorhergehenden Ansprüche 3 bis 7, wobei die Applanationslinse (9) durch die Stützstruktur (102) gehalten wird, die ihrerseits mit einem Grundkörper (101) gekoppelt ist, in dem der Laseremitter (113) mittels eines Verbindungselements (103) aufgenommen ist, so dass die durch die Applanationslinse (9), die Stützstruktur (102) und das Verbindungselement (103) gebildete Anordnung vor jeder Operation von dem Grundkörper (101) der Vorrichtung gelöst und sterilisiert werden kann.

9. Apparat nach Anspruch 8, wobei das Verbindungselement mit Kanälen und Steckverbindern versehen ist, um den Hauptkörper (101), in dem Zuführkanäle zum Zuführen von medizinischen Gasen oder Saugkanälen untergebracht sind, mit den chirurgischen Instrumenten (14, 21) fluidisch zu verbinden.

10. Apparat nach einem der vorhergehenden Ansprüche 1 bis 9, wobei die Fernsteuermittel eine Anzeige (203), auf der Bilder des Auges (1) projiziert werden, und Joystick-ähnliche Steuergeräte (104) umfassen.

11. Apparat nach einem der vorhergehenden Ansprüche 1 bis 9, wobei die Fernsteuermittel:
- eine Anzeige (203), auf der Bilder des Auges (1) projiziert werden, vom Operateur tragbare Handschuhe (206), die mit Drucksensoren (207) versehen sind, künstliche chirurgische Instrumente (209), die der Operateur mit den Handschuhen (206) halten kann, eine das Auge des Patienten simulierenden Modellpuppe (208) und
- eine Arbeitsfläche (202) in Kombination mit Sensoren ausgewählt aus Näherungssensoren des Füßchens gegenüber den Geweben des Patientenauges, Verformungssensoren der Gewebe, Temperatursensoren der Gewebe, Transparenzsensoren der Gewebe und Energieabgabe- und Absorptionssensoren der Gewebe, und
- Kameras zur Erfassung der Position der Hände und Finger des Operateurs gegenüber der Modellpuppe (208), wobei die Drucksensoren (207) den Druck erfassen, den der Operateur auf die künstliche chirurgische Instrumente (209) ausübt, und
- eine Steuereinheit, welche die von den Sensoren und den Kameras der Arbeitsfläche bereitgestellten Signale verarbeitet, um den Roboterarmen (13) und den chirurgischen Instrumenten (14, 21) die Bewegungen zu vermitteln, umfassen.

12. Apparat nach einem der vorhergehenden Ansprüche 1 bis 11, umfassend einen für die Transplantation bestimmten Behälter (300) für Okulargeweben (302), wobei der Behälter (300) mit einem Kulturmedium befüllbar ist und seinerseits umfasst:
- eine Mehrzahl von Pinzetten (303) oder auch eine die gleiche Funktion ausführende Stützfläche, die ausgebildet sind, das Okulargewebe (302) zurückzuhalten, so dass es sich gegenüber dem Behälter selbst nicht bewegen kann, und
- einen Deckel (304) zum dichtenden Verschließen des Behälters (300), so dass sein Innenmilieu steril bleibt,
wobei der Deckel (304) auch als Applanationsfläche wirkt und für Laserstrahlen transparent ist, wodurch ermöglicht wird, dass der Operateur das Okulargewebe (302) für die Transplantation ohne Öffnen des Behälters (300) und unter Vermeidung von Kontaminationen vorbereiten kann.

13. Apparat nach einem der vorhergehenden Ansprüche 1 bis 12, mit Mitteln zum Richten einer laminaren Strömung von sauberer und gefilterter Luft auf der Operationsstelle, im Bereich des Patientenauges oder des Füßchens des Roboterarms.

14. Apparat nach einem der vorhergehenden Ansprüche 1 bis 13, umfassend mindestens eine optische Faser zur Endobeleuchtung der Operationsstelle.

15. Apparat nach einem der vorhergehenden Ansprüche 1 bis 14, umfassend Sensoren, die die Instrumententemperatur und/oder die Vibrationen erfassen, denen die Instrumente ausgesetzt sind.

## Revendications

1. Un appareil (100) pour effectuer des opérations de chirurgie ophtalmique à distance, comprenant:
- un émetteur laser (113),
- des instruments chirurgicaux (14, 21),
- une structure de support (102) apte à positionner l'émetteur laser (113) par rapport à l'œil du patient (1), et
- des moyens de commande (104, 200, 206, 208, 209) pour commander à distance les instruments chirurgicaux (14, 21) et l'émetteur laser (113),
- au moins un bras robotique (13) également commandé à distance, à l'intérieur duquel l'instrument chirurgical (14) est logé télescopiquement et
en ce que le bras robotique (13) est pourvu d'un pied (17) appuyé sur l'œil du patient (1) et de moyens d'aspiration (18) maintenant, le cas échéant, le pied (17) adhérant à l'œil (1), par exemple lorsque l'instrument chirurgical (14) est inséré dans la chambre antérieure (5) de l'œil (1).

2. Appareil selon la revendication 1, dans lequel le pied (17) appuyé sur l'œil (1) définit, ensemble avec la surface (1) de l'œil lui-même, un site isolé du milieu environnant et en lui-même adapté pour effectuer des opérations chirurgicales.

3. Appareil selon la revendication 1 ou la revendication 2, comprenant en outre une lentille d'aplanation (9) et dans lequel la structure de support (102) est conçue pour positionner la lentille d'aplanation (9) sur l'œil (1) du patient et l'émetteur laser (113) par rapport à la lentille d'aplanation (9).

4. Appareil selon l'une quelconque des revendications 1-3, dans lequel les moyens d'aspiration (18) comprennent des canaux s'ouvrant au pied (17) et sont reliés à une pompe d'aspiration, qui est de préférence équilibrée de sorte que l'aspiration reste équilibrée par rapport à la pression interne de l'œil (1), de sorte que l'œil (1) ne se déforme pas et ne soit pas endommagé.

5. Appareil selon l'une quelconque des revendications 1-4, dans lequel des tiges de traction (16) adaptées pour transmettre des mouvements à l'instrument chirurgical (14), et/ou un conduit d'alimentation pour alimenter des fluides médicaux à l'instrument chirurgical (14), et/ou des conduits d'aspiration pour aspirer des tissus oculaires, par exemple des tissus d'une lentille cristalline (3) désintégrée par le laser, sont logés dans le bras robotique (13).

6. Appareil selon l'une quelconque des revendications précédentes 1-5, dans lequel le bras robotique (13) peut être rétracté de sorte que l'instrument chirurgical (14) puisse être laissé correctement dans l'œil (1).

7. Appareil selon l'une quelconque des revendications précédentes 1-6, comprenant en outre un circuit (106, 107, 108) pour faire circuler une solution saline équilibrée au niveau de l'œil (1), dans lequel la lentille d'aplanation (9), si présente, fait partie dudit circuit, la-même étant pourvue de canaux appropriés (110, 111).

8. Appareil selon l'une quelconque des revendications précédentes 3-7, dans lequel la lentille d'aplanation (9) est soutenue par la structure de support (102) qui, à son tour, est couplée à un corps principal (101) à l'intérieur duquel l'émetteur laser est logé (113), au moyen d'un élément de connexion (103), de sorte que l'ensemble formé par la lentille d'aplanation (9), la structure de support (102) et l'élément de connexion (103) puisse être libéré du corps principal (101) de l'appareil et stérilisé avant chaque opération.

9. Appareil selon la revendication 8, dans lequel l'élément de connexion est pourvu de canaux et de connecteurs de sorte à faire communiquer fluidiquement le corps principal (101), à l'intérieur duquel des conduits d'alimentation pour alimenter des gaz médicaux ou des conduits d'aspiration sont logés, avec les instruments chirurgicaux (14, 21).

10. Appareil selon l'une quelconque des revendications précédentes 1-9, dans lequel des moyens de commande à distance comprennent un écran (203), sur lequel des images de l'œil (1) sont projetées, et des instruments de commande de type joystick (104).

11. Appareil selon l'une quelconque des revendications précédentes 1-9, dans lequel les moyens de commande à distance comprennent:
- un écran (203) sur lequel des images de l'œil (1) sont projetées, des gants (206) que le chirurgien peut porter et pourvus de capteurs de pression (207), des faux instruments chirurgicaux (209) que le chirurgien manipule avec les gants (206), un mannequin (208) simulant l'œil du patient, et
- une surface de travail (202) associée avec des capteurs sélectionnés parmi des capteurs de proximité du pied par rapport aux tissus de l'œil du patient, des capteurs de déformations des tissus, des capteurs de température des tissus, des capteurs de transparence des tissus et des capteurs de libération et d'absorption d'énergie des tissus, et
- des caméras pour détecter la position des mains et des doigts du chirurgien par rapport au mannequin (208), dans lesquelles les capteurs de pression (207) détectent la pression appliquée par le chirurgien sur les faux instruments chirurgicaux (209), et
- une unité de commande traitant les signaux fournis par lesdits capteurs et les caméras de la surface de travail de sorte à transmettre les mouvements aux bras robotiques (13) et aux instruments chirurgicaux (14, 21).

12. Appareil selon l'une quelconque des revendications précédentes 1-11, comprenant un récipient (300) de tissus oculaires (302) destiné à la transplantation, dans lequel le récipient (300) peut être rempli d'un milieu de culture et comprend à son tour:
- un pluralité de pincettes (303) conçues pour retenir le tissu oculaire (302), ou bien une surface de support remplissant la même fonction, de sorte qu'elles ne puissent pas se déplacer par rapport au récipient lui-même, et
- un couvercle (304) pour fermer hermétiquement le récipient (300) de sorte que son environnement interne reste stérile,
dans lequel le couvercle (304) agit également comme une surface d'aplanation et est transparent aux faisceaux laser afin de permettre au chirurgien de préparer le tissu oculaire (302) à la transplantation sans ouvrir le récipient (300), de façon à éviter les contaminations.

13. Appareil selon l'une quelconque des revendications précédentes 1-12, comprenant des moyens pour diriger un flux laminaire d'air propre et d'air filtré sur le site chirurgical, au niveau de l'œil du patient ou du pied du bras robotique.

14. Appareil selon l'une quelconque des revendications précédentes 1-13, comprenant au moins une fibre optique pour l'endoillumination du site chirurgical.

15. Appareil selon l'une quelconque des revendications précédentes 1-14, comprenant des capteurs détectant la température de l'instrument et/ou les vibrations auxquelles les instruments sont soumis.
